# EUROPEAN PATENT SPECIFICATION

(11) **EP 3 733 160 B1**
(45) Date of publication and mention of the grant of the patent: **20.11.2024**
(21) Application number: 18895468.9
(22) Date of filing: 28.12.2018
(51) Int. Cl.: A61K 8/68, A61K 8/06, A61K 8/41, A61K 8/42, A61K 8/44, A61Q 19/00, A61K 8/36, A61K 8/49, A61K 8/63

(54) **EMULSIFIED COMPOSITION COMPRISING A HIGHLY CRYSTALLINE CERAMIDE**
EMULGIERTE ZUSAMMENSETZUNG ENTHALTEND EIN HOCHKRISTALLINES CERAMID
COMPOSITION ÉMULSIFIÉE COMPRENANT UNE CÉRAMIDE HAUTEMENT CRISTALLINE

(30) Priority: 28.12.2017 JP 2017254873
(43) Date of publication of application: 04.11.2020
(73) Proprietor: Kao Corporation, Tokyo 103-8210 (JP)
(72) Inventor: MIZOGUCHI, Keiko, Wakayama-shi, Wakayama 640-8580 (JP); KISHIMOTO, Yuko, Odawara-shi, Kanagawa 250-0002 (JP)
(74) Representative: Hoffmann Eitle
(86) International application number: PCT/JP2018/048403
(87) International publication number: WO 2019/131986

(56) References cited:
- EP-A1- 3 028 691
- EP-A2- 1 151 743
- JP-A- 2004 035 563
- JP-A- 2007 001 953
- JP-A- 2007 015 986
- JP-A- 2017 057 146
- JP-A- H1 171 239
- US-A1- 2005 031 570
- US-A1- 2014 194 522

## Description

### Field of the Invention

The present invention relates to an emulsified composition.

### Background of the Invention

Lipids called stratum corneum intercellular lipid are present in gaps defined between corneocytes constituting the stratum corneum, which is the outermost layer of skin. The lipid composition of stratum corneum intercellular lipid contains about 50% of ceramides, the rest including cholesterols, cholesterol esters, fatty acids and the like. Of these, ceramides are deeply involved with rough skin and dry skin, and it is known that the conditions of stratum corneum can be improved by externally supplementing ceramides.

For this point of view, various cosmetic products containing ceramides were reported. Ceramides have a high crystallinity and a high melting point and are hence difficult to be stabilized in a preparation. Thus, reports were made on a technique involving mixing a ceramide, phospholipid, and a cationic surfactant (Patent Literature 1), a technique involving adding a carboxylic acid and a base to ceramides to prepare an oil-in-water emulsion (Patent Literature 2), and an emulsified composition in which sphingosines, a glycerol mono-fatty acid ester, and a higher alcohol are added to ceramides (Patent Literature 3).

Patent Literature 4 describes a hair-treating agent containing (A) 0.01-50 wt. % of a specific iso type fatty acid cholesterol ester or a specific anti-iso type fatty acid cholesterol ester in a total amount of 0.01-50 wt. % based on the total amount of the final product and (B) at least one of a kind selected from sphingolipids (sphingoglucolipids such as glycosyl ceramide, and sphingophospholipids such as sphingomyelin) in an amount of 0.1-10 wt.%.

Patent Literature 5 concerns an anti-pro-oxidant glucosyl octyl ascorbic acid derivative.

Patent Literature 6 refers to an oil-in-water type emulsion cosmetic which contains (A) a hydrophilic surface active agent, (B) an oily component and (C) water, and weight ratio of the component (B) is 10 or more based on 1 of the component (A).

Patent Literature 7 describes an emulsion composition comprising the following ingredients (A), (B), (C), (D), (E), and (F): (A) glyceryl monofatty acid ester derived from a linear-chain fatty acid having 10 to 24 carbon atoms; (B) a higher alcohol having 10 to 24 carbon atoms; (C) a ceramide; (D) an anionic surfactant; (E) a polar oil selected from branched fatty acid esters having an IOB of from 0.2 to 0.85 and having a hydroxyl group or an amino group; and (F) water, wherein the mass ratio of the total content of ingredients (A), (B), and (C) and ingredient (D) in terms of acid to the content of ingredient (E), ((A)+(B)+(C)+(D))/(E), is from 1.2 to 25.

(Patent Literature 1) JP-A-2006-193464
(Patent Literature 2) JP-A-2006-312622
(Patent Literature 3) JP-A-2007-22997
(Patent Literature 4) JP H11 71239 A
(Patent Literature 5) JP 2017 057146 A
(Patent Literature 6) EP 1 151 743 A2
(Patent Literature 7) US 2014/194522 A1

### Summary of the Invention

The present invention provides an emulsified composition comprising (A) 0.01 mass% or more and 1 mass% or less of an anionic surfactant comprising one or more selected from the group consisting of N-acylamino acids, fatty acids having 12 to 24 carbon atoms, alkyl ether carboxylic acids, diacyl glutamic acid lysine, alkylsulfosuccinic acids, N-acyl methyl taurine, alkyl sulfates, polyoxyethylene alkyl ether sulfuric acids, polyoxyethylene alkyl ether phosphoric acids, and salts thereof, (B) a cationic surfactant comprising one or more selected from the group consisting of monoalkyl quaternary ammonium salts, dialkyl quaternary ammonium salts, diacylethyl hydroxyethylmonium methosulfates, and acyl arginine ethyl pyrrolidone carboxylic acid salts, (C) a ceramide, and (D) water, wherein the mass ratio of Component (A) to Component (B), A/B, is 0.5 or more and 60 or less.

### Detailed Description of the Invention

Stable addition of highly crystalline ceramides requires a large amount of surfactants, which, however, causes stickiness. Additionally, conventional emulsified compositions form a hydrophilic film by a surfactant and consequently the film runs down by water or sweat, posing a problem of poor film persistency.

Accordingly, the present invention provides an emulsified composition which contains a ceramide stably, has a smooth feel of use and has a good persistency of an applied film.

Under the circumstances, the present inventors conducted studies to obtain an emulsified composition which contains a ceramide stably, has a favorable feel of use and has a good persistency of an applied film, and found that when an anionic surfactant and a cationic surfactant are used in combination to emulsify an oil phase containing a ceramide with an aqueous phase, an emulsified composition can be obtained which provides a hydrophobic applied film to thereby bring about a smooth and firm feel of use, the applied film also having good persistency. Thus the present invention were accomplished.

The emulsified composition of the present invention contains a ceramide stably and provides an applied film with a smooth and firm feel, the applied film also having good persistency.

The emulsified composition of the present invention contains (A) 0.01 mass% or more and 1 mass% or less of an anionic surfactant comprising one or more selected from the group consisting of N-acylamino acids, fatty acids having 12 to 24 carbon atoms, alkyl ether carboxylic acids, diacyl glutamic acid lysine, alkylsulfosuccinic acids, N-acyl methyl taurine, alkyl sulfates, polyoxyethylene alkyl ether sulfuric acids, polyoxyethylene alkyl ether phosphoric acids, and salts thereof, (B) a cationic surfactant comprising one or more selected from the group consisting of monoalkyl quaternary ammonium salts, dialkyl quaternary ammonium salts, diacylethyl hydroxyethylmonium methosulfates, and acyl arginine ethyl pyrrolidone carboxylic acid salts, (C) a ceramide, and (D) water, wherein the mass ratio of Component (A) to Component (B), A/B, is 0.5 or more and 60 or less.

The anionic surfactant (A) contains one or more selected from the group consisting of carboxylic acid salt type such as N-acylamino acids, fatty acids having 12 to 24 carbon atoms, alkyl ether carboxylic acids, polyoxyethylene alkyl ether carboxylic acids, acyl lactic acids, N-acyl methylalanine, N-acyl sarcosine, diacyl amino acids, and salts thereof, sulfonic acid salt type such as alkanesulfonic acids, α-olefin sulfonic acids, α-sulfo fatty acid methyl esters, acyl isethionic acids, alkylsulfosuccinic acids, N-acyl methyl taurine, and salts thereof, sulfuric acid salt type such as alkyl sulfates, polyoxyethylene alkyl sulfates, alkyl ether sulfuric acids, polyoxyethylene alkyl ether sulfuric acids, fatty acid alkanolamide sulfates, and salts thereof, phosphoric acid salt type such as alkyl phosphates, polyoxyethylene alkyl ether phosphoric acids, and salts thereof. In view of improving a smooth and firm feel and the persistency of an applied film, the anionic surfactant (A) preferably contains one or more selected from the group consisting of N-acylamino acids, fatty acids, alkyl ether carboxylic acids, diacyl glutamic acid lysine, alkylsulfosuccinic acids, N-acyl methyl taurine, alkyl sulfates, polyoxyethylene alkyl sulfates, alkyl phosphates, polyoxyethylene alkyl ether phosphoric acids, and salts thereof.

Of these, the anionic surfactant (A) preferably contains one or more selected from the group consisting of N-acylamino acids, fatty acids having 12 to 24 carbon atoms, alkyl ether carboxylic acids, diacyl glutamic acid lysine, alkylsulfosuccinic acids, N-acyl methyl taurine, alkyl sulfates, polyoxyethylene alkyl sulfates, polyoxyethylene alkyl ether phosphoric acids, and salts thereof.

Further, the anionic surfactant (A) more preferably contains one or more selected from the group consisting of N-acylamino acids, fatty acids having 12 to 24 carbon atoms, alkyl ether carboxylic acids, diacyl glutamic acid lysine, alkylsulfosuccinic acids, N-acyl methyl taurine, polyoxyethylene alkyl sulfates, polyoxyethylene alkyl ether phosphoric aids, and salts thereof.

Additionally, the anionic surfactant (A) further preferably contains one or more selected from the group consisting of N-acylamino acids, fatty acids having 12 to 24 carbon atoms, diacyl glutamic acid lysine, N-acyl methyl taurine, polyoxyethylene alkyl ether phosphoric acids, and salts thereof, and furthermore preferably contains N-acylamino acids and salts thereof.

Examples of N-acylamino acids and salts thereof include N-acylglutamic acids such as N-lauroyl-L-glutamic acid, N-stearoyl-L-glutamic acid, N-myristoyl-L-glutamic acid, and salts thereof.

Examples of fatty acids having 12 to 24 carbon atoms include fatty acids such as lauric acid, palmitic acid, stearic acid, and salts thereof.

Examples of alkyl ether carboxylic acids and salts thereof include polyoxyethylene lauryl ether acetic acid and salts thereof.

Examples of diacyl glutamic acid lysine and salts thereof include dilauramidoglutamide lysine and salts thereof.

Examples of alkylsulfosuccinic acids and salts thereof include di-2-ethylhexyl sulfosuccinic acid and salts thereof.

Examples of N-acyl methyl taurine and a salt thereof include N-myristoyl-N-methyl taurine, N-lauroyl-N-methyl taurine, N-stearoyl-N-methyl taurine, and salts thereof.

Examples of alkyl sulfates and salts thereof include lauryl sulfate and salts thereof.

Examples of polyoxyethylene alkyl sulfates and salts thereof include polyoxyethylene lauryl sulfate and salts thereof.

Examples of alkyl phosphoric acids and salts thereof include monomyristyl phosphoric acid, monostearyl phosphoric acid, di(C12-15)pareth-8 phosphoric acids, and salts thereof.

Examples of polyoxyethylene alkyl phosphoric acids and salts thereof include polyoxyethylene oleyl ether phosphoric acid, polyoxyethylene cetyl ether phosphoric acid, polyoxyethylene stearyl ether phosphoric acid, and salts thereof.

Examples of the salt structure making up these salts include salts of alkali metal such as sodium and potassium, salts of basic amino acid such as L-arginine, L-histidine, and L-lysine, and alkanolamine salts such as triethanolamine. These anionic surfactants can be used singly or in combination of two or more thereof.

The content of Component (A) in the emulsified composition of the present invention is, in view of formability of a hydrophobic applied film and obtaining a smooth and firm feel and in view of improving the persistency of an applied film, 0.01 mass% or more, preferably 0.02 mass% or more, more preferably 0.05 mass% or more, and furthermore preferably 0.075 mass% or more, and 1 mass% or less, preferably 0.7 mass% or less, more preferably 0.5 mass% or less, and furthermore preferably 0.2 mass% or less. Specifically, the content of Component (A) is 0.01 mass% or more and 1 mass% or less, preferably 0.02 mass% or more and 0.7 mass% or less, more preferably 0.05 mass% or more and 0.5 mass% or less, and furthermore preferably 0.075 mass% or more and 0.2 mass% or less.

The cationic surfactant (B) contains one or more selected from the group consisting of those of aliphatic amine salt type, quaternary ammonium salt type, and DL-pyrrolidone carboxylic acid salt type, and, in view of improving a smooth and firm feel and improving the persistency of an applied film, the cationic surfactant (B) preferably contains quaternary ammonium salt and an acyl arginine ethyl pyrrolidone carboxylic acid salt.

Of these, the cationic surfactant (B) preferably contains one or more selected from the group consisting of monoalkyl quaternary ammonium salts, dialkyl quaternary ammonium salts, and acyl arginine ethyl pyrrolidone carboxylic acid salts.

Further, the cationic surfactant (B) further preferably contains one or more selected from the group consisting of mono C12-C24 alkyltrimethylammonium salts, di C12-C24 alkyldimethylammonium salts, diacylethyl hydroxyethylmonium methosulfates, and acyl arginine ethyl pyrrolidone carboxylic acid salts.

Examples of mono C12-C24 alkyltrimethylammonium salts include lauryltrimethylammonium chloride, stearyltrimethylammonium chloride, and behenyl trimethyl ammonium chloride.

Examples of di C12-C24 alkyldimethylammonium salts include dilauryldimethylammonium chloride, distearyldimethylammonium chloride, dibehenyldimethylammonium chloride.

Examples of diacylethyl hydroxyethylmonium methosulfates include dicocoylethyl hydroxyethylmonium methosulfate.

Examples of acyl arginine ethyl pyrrolidone carboxylic acid salts include cocoyl arginine ethyl pyrrolidone carboxylic acid salts.

These cationic surfactants can be used singly, or in combination of two or more thereof.

The content of Component (B) in the emulsified composition of the present invention is, in view of formability of a hydrophobic applied film and obtaining a smooth and firm feel and in view of improving the persistency of an applied film, preferably 0.001 mass% or more, more preferably 0.005 mass% or more, further preferably 0.01 mass% or more, and furthermore preferably 0.02 mass% or more, and preferably 0.5 mass% or less, more preferably 0.3 mass% or less, further preferably 0.1 mass% or less, furthermore preferably 0.05 mass% or less, and furthermore preferably 0.035 mass% or less. Specifically, the content of Component (B) is preferably 0.001 mass% or more and 0.5 mass% or less, more preferably 0.005 mass% or more and 0.3 mass% or less, further preferably 0.01 mass% or more and 0.1 mass% or less, furthermore preferably 0.01 mass% or more and 0.05 mass% or less, and furthermore preferably 0.02 mass% or more and 0.035 mass% or less.

Additionally, in the emulsified composition of the present invention, the mass ratio of Component (A) to Component (B), A/B, is, in view of formability of a hydrophobic applied film and obtaining a smooth and firm feel and in view of improving the persistency of an applied film, 0.5 or more, preferably 0.75 or more, more preferably 1 or more, further preferably 2 or more, and furthermore preferably 2.5 or more, and 60 or less, preferably 40 or less, more preferably 20 or less, further preferably 10 or less, and furthermore preferably 5 or less. Specifically, the mass ratio A/B is 0.5 or more and 60 or less, preferably 0.75 or more and 40 or less, more preferably 1 or more and 20 or less, further preferably 2 or more and 10 or less, and furthermore preferably 2.5 or more and 5 or less.

The ceramide (C) may be either a natural ceramide or a pseudo-ceramide, and preferably contains one or more selected from the group consisting of those represented by the following Formula (1) or (2).
(I) A natural ceramide represented by Formula (1) may be a naturally occurring ceramide or a synthetic substance having the same structure as of Formula (1): 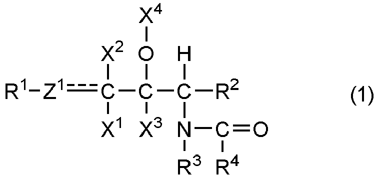 wherein R¹ represents a saturated or unsaturated linear, branched or cyclic hydrocarbon group having 7 to 19 carbon atoms and optionally substituted with a hydroxyl group; Z¹ represents a methylene group or a methine group; X¹, X², and X³ each independently represent a hydrogen atom, a hydroxyl group, or an acetoxy group; X⁴ represents a hydrogen atom or forms an oxo group together with an adjacent oxygen atom (provided that when Z¹ is a methine group, any one of X¹ and X² is a hydrogen atom, and the other is not present, and that when X⁴ forms an oxo group, X³ is not present); R² represents a hydroxymethyl group or an acetoxymethyl group; R³ represents a hydrogen atom or an alkyl group having 1 to 4 carbon atoms; R⁴ represents a saturated or unsaturated linear, branched or cyclic hydrocarbon group having 5 to 30 carbon atoms and optionally substituted with a hydroxyl group, or represents a group wherein a saturated or unsaturated linear or branched chain fatty acid having 8 to 22 carbon atoms and optionally substituted with a hydroxyl group is ester-bonded at the ω terminus of the alkyl group; and a dotted line represents an optional unsaturated bond.

Examples include compounds wherein R¹ is a linear alkyl group having preferably 7 to 19 carbon atoms, and further preferably 13 to 15 carbon atoms; and R⁴ is a linear alkyl group having 9 to 27 carbon atoms and optionally substituted with a hydroxyl group or a linear alkyl group having 9 to 27 carbon atoms to which linoleic acid is ester-bonded. Additionally, X⁴ preferably represents a hydrogen atom or forms an oxo group together with an oxygen atom. Particularly, R⁴ is preferably trichosyl, 1-hydroxypentadecyl, 1-hydroxytrichosyl, heptadecyl, 1-hydroxyundecyl, a nonacosyl group in which linoleic acid is ester-bonded at the ω terminus.

The natural ceramide is preferably one or more selected from the group consisting of ceramides of types 1 to 7, which are amidated sphingosine, dihydrosphingosine, phytosphingosine and sphingadienine (for example, swine and human ceramides described in Figure 2 of J. Lipid Res., 24:759 (1983) and Figure 4 of J. Lipid. Res., 35:2069 (1994).

N-Alkyl forms (for example, N-methyl form) of these ceramides are further included.

For these ceramides, a natural form (D(-) form) optically active substance may be used, a non-natural form (L(+) form) optically active substance may be used, and a mixture of natural and non-natural forms may be used. Relative configuration of the above compounds may be a natural form configuration, other non-natural form configurations, or a mixture thereof. Of these, one or more selected from the group consisting of compounds CERAMIDE1, CERAMIDE2, CERAMIDE3, CERAMIDE5 and CERAMIDE6II (all in INCI, 8th Edition) and those represented by the following formula are preferable.

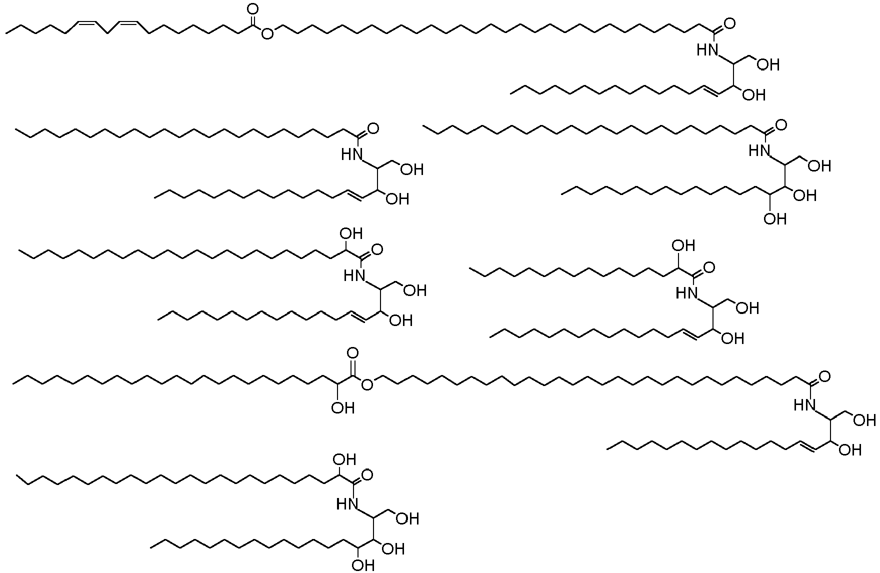

These may be a natural extract or a synthetic product, and a commercial product can be used.

When a commercial natural form ceramide is used, one or more selected from the group consisting of Ceramide I, Ceramide III, Ceramide IIIA, Ceramide IIIB, Ceramide IIIC, Ceramide VI (all from COSMO FARM), Ceramide TIC-001 (TAKASAGO INTERNATIONAL CORPORATION), CERAMIDE II (Quest International), DS-Ceramide VI, DS-CLA-Phytoceramide, C6-Phytoceramide, DS-ceramide Y3S (Doosan Corporation), and CERAMIDE2 (Sederma) are preferable.

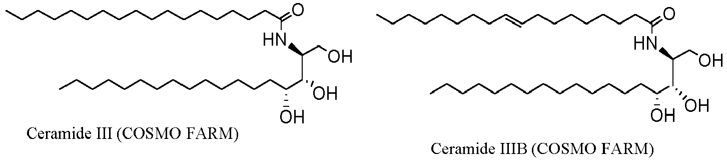

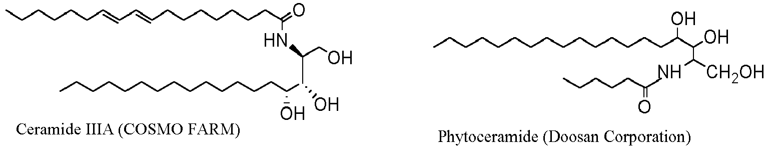

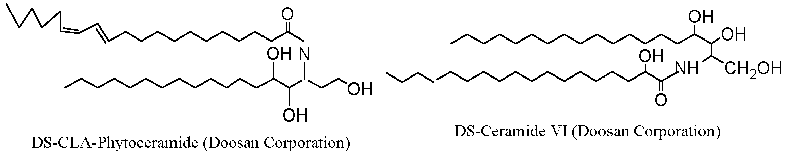

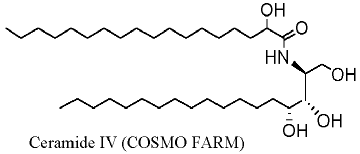

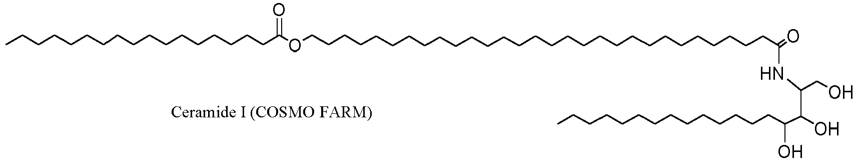

### (II) A pseudo-ceramide represented by Formula (2):

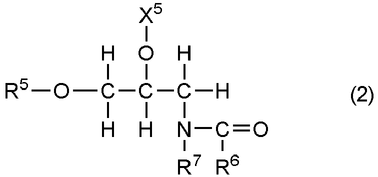

wherein R⁵ represents a saturated or unsaturated linear, branched or cyclic hydrocarbon group having 10 to 22 carbon atoms and optionally substituted with a hydroxyl group, or a hydrogen atom; X⁵ represents a hydrogen atom, an acetyl group, or a glyceryl group; R⁶ is a saturated or unsaturated linear, branched or cyclic hydrocarbon group having 5 to 22 carbon atoms and optionally substituted with a hydroxyl group or an amino group, or represents a group wherein a saturated or unsaturated linear or branched chain fatty acid having 8 to 22 carbon atoms and optionally substituted with a hydroxyl group is ester-bonded at the ω terminus of the hydrocarbon group; and R⁷ represents a hydrogen atom, or represents an alkyl group having the total carbon atom of 1 to 30 and optionally substituted with a hydroxyl group, a hydroxyalkoxy group, an alkoxy group, or an acetoxy group.

R⁶ is particularly preferably nonyl, tridecyl, pentadecyl, an undecyl group in which linoleic acid is ester-bonded at the ω-position, a pentadecyl group in which linoleic acid is ester-bonded at the ω-position, a pentadecyl group in which 12-hydroxystearic acid is ester-bonded at the ω-position, or an undecyl group in which methyl-branched isostearic acid is amide-bonded at the ω-position.

When R⁵ is a hydrogen atom, R⁷ is preferably an alkyl group having the total carbon atom of 10 to 30, preferably the total carbon atom of 12 to 20, in which a hydroxyl group, a hydroxyalkoxy group, an alkoxy group, or an acetoxy group is optionally substituted, and when R⁵ is a saturated or unsaturated linear, branched or cyclic hydrocarbon group having 10 to 22 carbon atoms and optionally substituted with a hydroxyl group, R⁷ preferably represents a hydrogen atom, or an alkyl group having the total carbon atom of 1 to 8 and optionally substituted with a hydroxyl group, a hydroxyalkoxy group, an alkoxy group, or an acetoxy group. The hydroxyalkoxy group or the alkoxy group of R⁷ preferably has 1 to 7 carbon atoms.

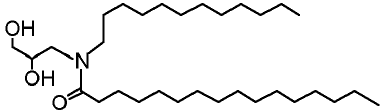

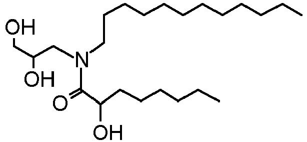

Formula (2) is preferably at least one selected from the group consisting of pseudo-ceramides wherein R⁵ is a hexadecyl group, X⁵ is an hydrogen atom, R⁶ is a pentadecyl group, and R⁷ is a hydroxyethyl group; and those wherein R⁵ is a hexadecyl group, X⁵ is a hydrogen atom, R⁶ is a nonyl group, and R⁷ is a hydroxyethyl group, and further preferably those of Formula (2) wherein R⁵ is a hexadecyl group, X⁵ is a hydrogen atom, R⁶ is a pentadecyl group, R⁷ is a hydroxyethyl group (N-(hexadecyloxyhydroxypropyl)-N-hydroxyethylhexadecanamide.

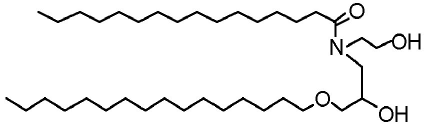

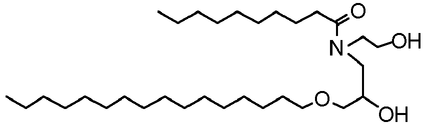

The content of Component (C) in the emulsified composition of the present invention is, in view of obtaining a moisturizing effect and a smooth and firm feel and in view of improving the persistency of an applied film, preferably 0.0005 mass% or more, more preferably 0.001 mass% or more, further preferably 0.01 mass% or more, furthermore preferably 0.1 mass% or more, furthermore preferably 0.5 mass% or more, and furthermore preferably 0.8 mass% or more, and preferably 50 mass% or less, more preferably 30 mass% or less, further preferably 20 mass% or less, and furthermore preferably 10 mass% or less. Specifically, the content of Component (C) is preferably 0.0005 mass% or more and 50 mass% or less, more preferably 0.001 mass% or more and 30 mass% or less, further preferably 0.01 mass% or more and 20 mass% or less, furthermore preferably 0.1 mass% or more and 10 mass% or less, furthermore preferably 0.5 mass% or more and 10 mass% or less, and furthermore preferably 0.8 mass% or more and 10 mass% or less.

Additionally, in view of formability of a hydrophobic applied film and obtaining a smooth and firm feel and in view of improving the persistency of an applied film, the mass ratio of the total amount of Component (A) and Component (B) to the amount of Component (C), (A+B)/C, in the emulsified composition of the present invention is preferably 0.002 or more, more preferably 0.005 or more, further preferably 0.01 or more, furthermore preferably 0.05 or more, and furthermore preferably 0.1 or more, and preferably 5 or less, more preferably 3 or less, further preferably 1 or less, furthermore preferably 0.3 or less, and furthermore preferably 0.2 or less. Specifically, it is preferably 0.002 or more and 5 or less, more preferably 0.005 or more and 3 or less, further preferably 0.01 or more and 1 or less, furthermore preferably 0.05 or more and 0.3 or less, and furthermore preferably 0.1 or more and 0.2 or less.

The content of (D) water in the emulsified composition of the present invention is, in view of obtaining a smooth and firm feel and in view of improving the persistency of an applied film, preferably 10 mass% or more and 99.5 mass% or less, and more preferably 40 mass% or more and 99 mass% or less.

The emulsified composition of the present invention preferably contains (E) one or more selected from the group consisting of cholesterol and cholesterol fatty acid esters in addition to the above Components in view of stably incorporating the ceramide (C) thereinto, formability of a hydrophobic applied film, obtaining a smooth and firm feel, and improving the persistency of an applied film.

The cholesterol fatty acid ester is preferably a cholesterol ester with a fatty acid having 12 to 24 carbon atoms. More specifically, it is preferable to be one or more selected from the group consisting of cholesteryl laurate, cholesteryl palmitate, cholesteryl myristate, cholesteryl oleate, cholesteryl isostearate, and cholesteryl linoleate.

The content of Component (E) in the emulsified composition is, in view of formability of a hydrophobic applied film, a smooth and firm feel, and improving the persistency of an applied film, preferably 0.001 mass% or more and 10 mass% or less, more preferably 0.05 mass% or more and 5 mass% or less, further preferably 0.01 mass% or more and 3 mass% or less, and furthermore preferably 0.1 mass% or more and 1 mass% or less.

The emulsified composition of the present invention can further contain (F) a base and/or (G) an acid in addition to Components (A) and (B) to adjust the pH.

The base (F) is not particularly limited and may be an organic base or an inorganic base.

The organic base is preferably one or more selected from the group consisting of basic amino acids and alkanolamines. Specifically, it is preferable to be one or more selected from the group consisting of basic amino acids such as L-arginine, lysine, and histidine; and alkanolamines such as monoethanolamine, diethanolamine, triethanolamine, aminomethyl propanol, aminomethyl propanediol, aminoethyl propanediol and trishydroxymethylaminoethane.

The inorganic base is preferably one or more selected from the group consisting of calcium hydroxide, sodium hydroxide, and potassium hydroxide.

Of these, the base is preferably one or more selected from the group consisting of L-arginine, calcium hydroxide, sodium hydroxide, and potassium hydroxide.

These for the base (F) can be used singly or in combination of two or more thereof, and is contained in an amount of preferably from 0 to 10 mass%, and more preferably from 0.001 to 5 mass%, in the whole composition in view of achieving a further favorable feel of use.

Additionally, the acid (G) may be an organic acid or an inorganic acid.

The organic acid is preferably one or more selected from the group consisting of monocarboxylic acids, dicarboxylic acids, oxycarboxylic acids, and acidic amino acids. Specifically, it is preferably one or more selected from the group consisting of monocarboxylic acids such as acetic acid, propionic acid, and butyric acid; dicarboxylic acids such as succinic acid, phthalic acid, fumaric acid, oxalic acid, malonic acid, glutaric acid, and adipic acids; oxycarboxylic acids such as glycolic acid, citric acid, lactic acid, pyruvic acid, malic acid, and tartaric acid; and acidic amino acids such as glutamic acid, and aspartic acid.

The inorganic acid is preferably one or more selected from the group consisting of hydrochloric acid, nitric acid, nitrous acid, sulfuric acid, sulfurous acid, phosphoric acid, phosphonic acid, and phosphinic acid.

Of these, the acid is preferably one or more selected from the group consisting of acidic amino acids and phosphoric acid.

One or more of these can be used as the acid (G), and the acid (G) is preferably contained in an amount of from 0 to 10 mass%, and more preferably from 0.001 to 5 mass%, based on the whole composition.

According to the present invention, the effects of the present invention, which are the formation of a hydrophobic applied film, a smooth and firm feel of use and the persistency of such a film, can be obtained in wide pH ranges (pH 4 to 8).

The emulsified composition of the present invention can be produced, for example, by heating and mixing an oil phase containing Components (A) to (C) at from 80 to 95°C, adding an aqueous phase component(s) thereto and stirring the resulting mixture to homogeneously emulsify.

The method for producing the emulsified composition of the present invention specifically includes Step 1 of heating and mixing an oil phase containing Components (A) to (C), and Step 2 of mixing an aqueous phase containing Component (D) with the oil phase and stirring the resultant. Further, the method preferably includes, between Step 1 and Step 2, Step 12 of dissolving Component (F) or Component (G) in a part of Component (D) to obtain an aqueous solution and adding the aqueous solution to the oil phase to obtain a mixture, and Step 13 of cooling the mixture after Step 12. Additionally, the production method can include, after Step 2, Step 2-2 of dissolving Component (F) or Component (G) in a part of Component (D) to obtain an aqueous solution and adding the aqueous solution to the composition to obtain a mixture. In these cases, the content of Component (D) is the total amount of the amounts added at each Step. Additionally, in the step of heating and mixing, the heating temperature is preferably at the highest melting point of the melting points of the components to be mixed or more and 100°C or less.

The method for producing the emulsified composition of the present invention preferably includes Step 1 of heating and mixing an oil phase containing Components (A) to (C), and preferably Component (E), Step 12 of dissolving Component (F) or Component (G) in a part of Component (D) to obtain an aqueous solution and adding the aqueous solution to the oil phase to obtain a mixture, Step 13 of cooling the mixture after Step 12, and Step 2 of further mixing an aqueous phase containing remaining of Component (D) with the oil phase and stirring the resultant.

The emulsified composition of the present invention can use a commercial homomixer, dispermixer, ultramixer, high pressure homogenizer, or micromixer but the high pressure homogenizer and the micromixer can be used as a means for further micronization.

The emulsification method using the high pressure homogenizer or the micromixer is preferable in view of uniformalizing particle sizes of emulsified particles, improving storage stability, and the expectation in improved permeability of micronized ceramides to the skin, and can produce a micronized emulsion having a particle size of dispersed particles (volume average particle size) of 200 nm or less, preferably 100 nm or less, and more preferably 50 nm or less.

The emulsified composition of the present invention has a vesicle formed and the vesicle structure is stable. Additionally, when the emulsified composition of the present invention is applied to the skin, a highly hydrophobic applied film is formed. The formation of such a highly hydrophobic applied film provides a high moisturizing function and also a smooth and firm feel. Further, a highly hydrophobic applied film shows strong water resistance thus making a favorable feel of use last.

The emulsified composition of the present invention is applicable as an external preparation for the skin such as cosmetic products. In the case of a cosmetic product, the composition can further contain, in addition to the above components, components typically used for cosmetic products such as an oil component other than above, a lower alcohol, a moisturizer, an antioxidant, a preservative, a chelating agent, a whitening agent, a UV absorber, vitamins, a plant extract, other various medicinal components, a powder, a perfume, and a coloring material.

With reference to embodiments described above, the present invention further discloses the following composition.

An emulsified composition comprising (A) 0.01 mass% or more and 1 mass% or less of an anionic surfactant comprising one or more selected from the group consisting of N-acylamino acids, fatty acids having 12 to 24 carbon atoms, alkyl ether carboxylic acids, diacyl glutamic acid lysine, alkylsulfosuccinic acids, N-acyl methyl taurine, alkyl sulfates, polyoxyethylene alkyl ether sulfuric acids, polyoxyethylene alkyl ether phosphoric acids, and salts thereof, (B) a cationic surfactant comprising one or more selected from the group consisting of monoalkyl quaternary ammonium salts, dialkyl quaternary ammonium salts, diacylethyl hydroxyethylmonium methosulfates, and acyl arginine ethyl pyrrolidone carboxylic acid salts, (C) a ceramide, and (D) water, wherein the mass ratio of Component (A) to Component (B), A/B, is 0.5 or more and 60 or less.

The anionic surfactant (A) may comprise one or more selected from the group consisting of N-acylamino acids, fatty acids having 12 to 24 carbon atoms, diacyl glutamic acid lysine, N-acyl methyl taurine, polyoxyethylene alkyl ether phosphoric acids, and salts thereof.

The anionic surfactant (A) may comprise one or more selected from the group consisting of N-acylamino acids and salts thereof.

The content of Component (A) is preferably 0.02 mass% or more, further preferably 0.05 mass% or more, and furthermore preferably 0.075 mass% or more, and preferably 0.7 mass% or less, further preferably 0.5 mass% or less, and furthermore preferably 0.2 mass% or less, and preferably 0.02 mass% or more and 0.7 mass% or less, further preferably 0.05 mass% or more and 0.5 mass% or less, and furthermore preferably 0.075 mass% or more and 0.2 mass% or less.

The cationic surfactant (B) may comprise one or more selected from the group consisting of mono C12-C24 alkyltrimethylammonium salts, di C12-C24 alkyldimethylammonium salts, diacylethyl hydroxyethylmonium methosulfates, and acyl arginine ethyl pyrrolidone carboxylic acid salts.

The content of Component (B) is preferably 0.001 mass% or more, more preferably 0.005 mass% or more, further preferably 0.01 mass% or more, and furthermore preferably 0.02 mass% or more, and preferably 0.5 mass% or less, more preferably 0.3 mass% or less, further preferably 0.1 mass% or less, furthermore preferably 0.05 mass% or less, and furthermore preferably 0.035 mass% or less, and preferably 0.001 mass% or more and 0.5 mass% or less, more preferably 0.005 mass% or more and 0.3 mass% or less, further preferably 0.01 mass% or more and 0.1 mass% or less, furthermore preferably 0.01 mass% or more and 0.05 mass% or less, and furthermore preferably 0.02 mass% or more and 0.035 mass% or less.

The ceramide (C) may comprise one or more selected from the group consisting of natural ceramides and pseudo-ceramides.

The content of Component (C) is preferably 0.0005 mass% or more, more preferably 0.001 mass% or more, further preferably 0.01 mass% or more, furthermore preferably 0.1 mass% or more, furthermore preferably 0.5 mass% or more, and furthermore preferably 0.8 mass% or more, and preferably 50 mass% or less, more preferably 30 mass% or less, further preferably 20 mass% or less, furthermore preferably 10 mass% or less, and preferably 0.0005 mass% or more and 50 mass% or less, more preferably 0.001 mass% or more and 30 mass% or less, further preferably 0.01 mass% or more and 20 mass% or less, furthermore preferably 0.1 mass% or more and 10 mass% or less, furthermore preferably 0.5 mass% or more and 10 mass% or less, and furthermore preferably 0.8 mass% or more and 10 mass% or less.

The mass ratio of Component (A) to Component (B), A/B, is preferably 1 or more, more preferably 2 or more, further preferably 2.5 or more, and preferably 40 or less, more preferably 20 or less, further preferably 10 or less, furthermore preferably 5 or less, and preferably 0.75 or more and 40 or less, more preferably 1 or more and 20 or less, further preferably 2 or more and 10 or less, and furthermore preferably 2.5 or more and 5 or less.

The mass ratio of the total amount of Component (A) and Component (B) to the amount of Component (C), (A+B)/C, is preferably 0.002 or more, more preferably 0.005 or more, further preferably 0.01 or more, furthermore preferably 0.05 or more, furthermore preferably 0.1 or more, and preferably 5 or less, more preferably 3 or less, further preferably 1 or less, further preferably 0.3 or less, and furthermore preferably 0.2 or less, and preferably 0.002 or more and 5 or less, more preferably 0.005 or more and 3 or less, further preferably 0.01 or more and 1 or less, furthermore preferably 0.05 or more and 0.3 or less, and furthermore preferably 0.1 or more and 0.2 or less.

The the content of Component (D) is preferably 10 mass% or more and 99.5 mass% or less, and more preferably 40 mass% or more and 99 mass% or less.

The emulsified composition may further comprise (E) one or more selected from the group consisting of cholesterol and fatty acid cholesterol esters.

The emulsified composition may further comprise (F) a base and/or (G) an acid.

The emulsified composition may be a cosmetic product.

A method for producing the emulsified composition, comprising Step 1 of heating and mixing an oil phase comprising Components (A) to (C), Step 12 of dissolving the base as Component (F) or the acid as Component (G) in a part of water as Component (D) to obtain an aqueous solution and adding the aqueous solution to the oil phase to obtain a mixture, Step 13 of cooling the mixture after Step 12, and Step 2 of mixing an aqueous phase comprising remaining of Component (D) with the oil phase and stirring the resultant.

The oil phase in Step 1 may further comprise (E) one or more selected from the group consisting of cholesterol and fatty acid cholesterol esters, and the oil phase comprising Components (A) to (C) and Component (E) may be heated and mixed in Step 1.

### Example

The present invention is further described with reference to examples.

### (Production Method)

Components (A), (B), (C), and (E) were mixed, dissolved by heating at from 80 to 95°C and stirred. Further, an aqueous solution in which (G) phosphoric acid or (F) L-arginine had been dissolved in a part (10 mass%) of water was added thereto, and the resultant was homogenized, and subsequently cooled to 25°C while stirring. Then, remaining components were mixed therewith and homogenized by stirring.

### (Evaluation method)

### (1) Condition of emulsified particles

Condition of the emulsified particles was observed immediately after a sample was prepared for the presence or absence of particles and Maltese cross using a microscope (under direct light and polarized light). Samples in which particles and Maltese cross were observed ranked as "A", samples in which a multilayer lamellar structure of particles was observed ranked as "B", and samples in which no particle was observed ranked as "C".

### (2) Feel of an applied film

Panelists (N=3) specialized in cosmetic product evaluation applied a proper amount (0.3 g) of a sample on back of the hand and evaluated (total 12 points) on a feel (smooth and firm feel) by criteria with 4 ratings (4 points: Good, 3 points: Rather good, 2 points: Rather not good, 1 point: Not good). Total points are shown in the table.

### (3) Persistency of an applied film (after washed)

A proper amount (0.3 g) of a sample was applied on back of the hand to form a dry applied film on the skin, subsequently the hand was washed with water, and then a feel (smooth and firm feel) was examined. Cases in which the feel persisted ranked as "A", cases in which the feel rather persisted ranked as "B", and cases in which the feel did not persist ranked as "C".

### (4) Contact angle measurement

0.5 g of a sample was applied to on per area of 2.5 cm x 2.5 cm of an artificial leather and dried, and subsequently reapplication was repeatedly carried out 6 times. After drying for 24 hours, 1 µL of a water droplet was dropped on the applied film under environment at 25°C and contact angles immediately after dropping and 60 seconds after dropping were measured using an automatic contact angle meter DM-501H1 (manufactured by Kyowa Interface Science Co., Ltd./Kitahama factory).

### (5) Hydrophilicity/Hydrophobicity of an applied film

Contact angles immediately after dropping and 60 seconds after dropping greater than 30° were defined as hydrophobic, whereas those smaller than 30° were defined as hydrophilic.

### Example 1 and Comparative Examples 1, 2

Comparison was made between a case in which an anionic surfactant and a cationic surfactant were used together with a ceramide and a case in which an anionic surfactant or a cationic surfactant was singly used with a ceramide. The results revealed that, when an anionic surfactant and a cationic surfactant were used together, a hydrophobic applied film was formed, a smooth and firm feel of use was obtained, and the film persisted, as evident in Table 1.

**[Table 1]**

| Component | Example | Comparative Example | | |
|---|---|---|---|---|
| | 1 | 1 | 2 | |
| (A) N-Stearoyl-L-glutamic acid | 0.075 | 0.1 | - | |
| (B) Distearyldimethylammonium chloride | 0.025 | - | 0.1 | |
| (C) Pseudo-ceramide* 1 | 1.0 | 1.0 | 1.0 | |
| (E) Cholesterol | 0.25 | 0.25 | 0.25 | |
| (G) Phosphoric acid | 0.04 | 0.04 | 0.04 | |
| (D) Purified water | 98.61 | 98.61 | 98.61 | |
| Total | 100 | 100 | 100 | |
| Evaluation results | | | | |
| (1) Emulsification condition | | A | A | B |
| (2) Feel of applied film (smooth and firm feel) | | 12 | 5 | 3 |
| (3) Persistency of applied film (after washing) | | A | C | C |
| (4) Contact angle (°) | Immediately after dropping | 80 | 26 | 62 |
| | 60 Seconds after dropping | 60 | 15 | 23 |
| (5) Hydrophilicity/hydrophobicity of applied film | | Hydrophobic | Hydrophilic | Hydrophilic |

| | | | | |
|---|---|---|---|---|
| *1: N-(Hexadecyloxyhydroxypropyl)-N-hydroxyethylhexadecanamide | | | | |

### Examples 2 to 7

Contents of the anionic surfactant and the cationic surfactant were changed and the evaluations described above were carried out. The results reveal that the effects of the present invention, which are the formation of a hydrophobic applied film, a smooth and firm feel of use and the persistency of the film, were obtained regardless of these contents, but when A/B is 0.75 or more and 40 or less, the evaluation results are favorable.

**[Table 2]**

| Component | | Example | | | | | | |
|---|---|---|---|---|---|---|---|---|
| | | 1 | 2 | 3 | 4 | 5 | 6 | 7 |
| (A) N-Stearoyl-L-glutamic acid | | 0.075 | 0.1 | 0.3 | 1.0 | 0.075 | 0.075 | 0.075 |
| (B) Distearyldimethylammonium chloride | | 0.025 | 0.025 | 0.025 | 0.025 | 0.01 | 0.05 | 0.1 |
| (C) Pseudo-ceramide*1 | | 1.0 | 1.0 | 1.0 | 1.0 | 1.0 | 1.0 | 1.0 |
| (E) Cholesterol | | 0.25 | 0.25 | 0.25 | 0.25 | 0.25 | 0.25 | 0.25 |
| (G) Phosphoric acid | | 0.04 | - | - | - | 0.016 | 0.08 | 0.16 |
| (F) L-Arginine | | - | 0.3 | 0.9 | 3.0 | - | - | - |
| (D) Purified water | | 98.61 | 98.325 | 97.525 | 94.725 | 98.649 | 98.545 | 98.415 |
| Total | | 100 | 100 | 100 | 100 | 100 | 100 | 100 |
| (A/B) | | 3 | 4 | 12 | 40 | 7.5 | 1.5 | 0.75 |
| ((A+B)/C) | | 0.1 | 0.125 | 0.325 | 1.025 | 0.085 | 0.125 | 0.175 |

| Evaluation results | | | | | | | | |
|---|---|---|---|---|---|---|---|---|
| (1) Emulsification condition | | A | A | B | B | B | A | A |
| (2) Feel of applied film (smooth and firm feel) | | 12 | 12 | 11 | 9 | 12 | 10 | 9 |
| (3) Persistency of applied film (after washing) | | A | A | A | A | A | A | A |
| (4) Contact angle (°) | Immediately after dropping | 80 | 80 | 82 | 52 | 82 | 77 | 77 |
| | 60 Seconds after dropping | 60 | 60 | 62 | 41 | 62 | 48 | 48 |
| (5) Hydrophilicity/hydrophobicity of applied film | | Hydrophobic | Hydrophobic | Hydrophobic | Hydrophobic | Hydrophobic | Hydrophobic | Hydrophobic |

| | | | | | | | | |
|---|---|---|---|---|---|---|---|---|
| *1: N-(Hexadecyloxyhydroxypropyl)-N-hydroxyethylhexadecanamide | | | | | | | | |

### Examples 8 to 12

The evaluations described above were carried out using various anionic surfactants and cationic surfactants. The results revealed that the effects of the present invention, which are the formation of a hydrophobic applied film, a smooth and firm feel of use and the persistency of the film, were obtained regardless of the type of anionic surfactants and cationic surfactants, as evident in Table 3.

**[Table 3]**

| Component | | Example | | | | | |
|---|---|---|---|---|---|---|---|
| | | 1 | 8 | 9 | 10 | 11 | 12 |
| (A) N-Stearoyl-L-glutamic acid | | 0.075 | 0.075 | 0.075 | - | - | - |
| (A) Sodium dilauramidoglutamide lysine (act.30%) | | - | - | - | 0.23 | - | - |
| (A) Di(C12-C15)pareth-8-phosphoric acid | | - | - | - | - | 0.075 | - |
| (A) Stearic acid | | - | - | - | - | - | 0.075 |
| (B) Distearyldimethylammonium chloride | | 0.025 | - | - | - | - | 0.025 |
| (B) Monobehenylammonium chloride | | - | - | - | - | 0.025 | - |
| (B) Cocoyl arginine ethyl PCA | | - | 0.025 | - | 0.025 | - | - |
| (B) Dicocoylethyl hydroxyethylmonium methosulfate | | - | - | 0.025 | - | - | - |
| (C) Pseudo-ceramide*1 | | 1.0 | 1.0 | 1.0 | 1.0 | 1.0 | 1.0 |
| (E) Cholesterol | | 0.25 | 0.25 | 0.25 | 0.25 | 0.25 | 0.25 |
| (G) Phosphoric acid | | 0.04 | 0.04 | 0.04 | 0.04 | 0.04 | 0.04 |
| (D) Purified water | | 98.61 | 98.61 | 98.61 | 98.455 | 98.61 | 98.61 |
| Total | | 100 | 100 | 100 | 100 | 100 | 100 |
| (A/B) | | 3 | 3 | 3 | 2.76 | 3 | 3 |
| ((A+B)/C) | | 0.1 | 0.1 | 0.1 | 0.094 | 0.1 | 0.1 |

| Evaluation results | | | | | | | |
|---|---|---|---|---|---|---|---|
| (1) Emulsification condition | | A | A | A | A | A | A |
| (2) Feel of applied film (smooth and firm feel) | | 12 | 10 | 12 | 9 | 10 | 8 |
| (3) Persistency of applied film (after washing) | | A | A | A | A | A | A |
| (4) Contact angle (°) | Immediately after dropping | 80 | 70 | 105 | 100 | 85 | 105 |
| | 60 Seconds after dropping | 60 | 40 | 90 | 60 | 50 | 50 |
| (5) Hydrophilicity/hydrophobicity of applied film | | Hydrophobic | Hydrophobic | Hydrophobic | Hydrophobic | Hydrophobic | Hydrophobic |

| | | | | | | | |
|---|---|---|---|---|---|---|---|
| *1: N-(Hexadecyloxyhydroxypropyl)-N-hydroxyethylhexadecanamide | | | | | | | |

### Examples 13 to 16

The emulsified compositions having the compositions of Table 4 were obtained and the evaluations described above were carried out. The results revealed that the effects of the present invention, which are the formation of a hydrophobic applied film, a smooth and firm feel of use and the persistency of the film, were obtained regardless of the ceramide content, as evident in Table 4. Additionally, it was revealed that the effects of the present invention, which are the formation of a hydrophobic applied film, a smooth and firm feel of use and the persistency of the film, were obtained in wide pH ranges regardless of pH.

**[Table 4]**

| Component | | Example | | | | |
|---|---|---|---|---|---|---|
| | | 1 | 13 | 14 | 15 | 16 |
| (A) N-Stearoyl-L-glutamic acid | | 0.075 | 0.075 | 0.075 | 0.075 | 0.075 |
| (B) Distearyldimethylammonium chloride | | 0.025 | 0.025 | 0.025 | 0.025 | 0.025 |
| (C) Pseudo-ceramide*1 | | 1.0 | 1.0 | 0.5 | 10.0 | 1.0 |
| (E) Cholesterol | | 0.25 | - | 0.25 | 0.25 | 0.25 |
| (G) Phosphoric acid | | 0.04 | 0.04 | 0.04 | 0.04 | - |
| (F) L-arginine | | - | - | - | - | 0.225 |
| (D) Purified water | | 98.61 | 98.86 | 99.11 | 89.61 | 98.425 |
| Total | | 100 | 100 | 100 | 100 | 100 |
| (A/B) | | 3 | 3 | 3 | 3 | 3 |
| ((A+B)/C) | | 0.1 | 0.1 | 0.2 | 0.01 | 0.1 |
| pH | | 4 | - | - | - | 8 |

| Evaluation results | | | | | | |
|---|---|---|---|---|---|---|
| (1) Emulsification condition | | A | A | A | A | B |
| (2) Feel of applied film (smooth and firm feel) | | 12 | 10 | 12 | 12 | 10 |
| (3) Persistency of applied film (after washing) | | A | A | A | A | A |
| (4) Contact angle (°) | Immediately after dropping | 80 | 90 | 82 | 102 | 80 |
| | 60 Seconds after dropping | 60 | 68 | 46 | 88 | 50 |
| (5) Hydrophilicity/hydrophobicity of applied film | | Hydrophobic | Hydrophobic | Hydrophobic | Hydrophobic | Hydrophobic |

| | | | | | | |
|---|---|---|---|---|---|---|
| *1: N-(Hexadecyloxyhydroxypropyl)-N-hydroxyethylhexadecanamide | | | | | | |

### Comparative Example 3

The emulsified compositions having the compositions of Table 5 were obtained and the evaluations described above were carried out. The results revealed that the emulsified composition of Comparative Example 3, in which the mass ratio of Component (A) to Component (B), A/B, exceeded 60, failed to form a hydrophobic applied film and the film had poor persistency, as evident in Table 5.

**[Table 5]**

| Component | | Example | Comparative Example |
|---|---|---|---|
| | | 4 | 3 |
| (A) N-Stearoyl-L-glutamic acid | | 1.0 | 1.0 |
| (B) Distearyldimethylammonium chloride | | 0.025 | 0.01 |
| (C) Pseudo-ceramide*1 | | 1.0 | 1.0 |
| (E) Cholesterol | | 0.25 | 0.25 |
| (G) Phosphoric acid | | - | - |
| (F) L-arginine | | 3.0 | 3.0 |
| (D) Purified water | | 94.725 | 94.74 |
| Total | | 100 | 100 |
| (A/B) | | 40 | 100 |
| ((A+B)/C) | | 1.025 | 1.01 |

| Evaluation results | | | |
|---|---|---|---|
| (1) Emulsification condition | | B | B |
| (2) Feel of applied film (smooth and firm feel) | | 9 | 7 |
| (3) Persistency of applied film (after washing) | | A | B |
| (4) Contact angle (°) | Immediately after dropping | 52 | 43 |
| | 60 Seconds after dropping | 41 | 29 |
| (5) Hydrophilicity/hydrophobicity of applied film | | Hydrophobic | Hydrophilic |

| | | | |
|---|---|---|---|
| * 1: N-(Hexadecyloxyhydroxypropyl)-N-hydroxyethylhexadecanamide | | | |

## Claims

1. An emulsified composition comprising (A) 0.01 mass% or more and 1 mass% or less of an anionic surfactant comprising one or more selected from the group consisting of N-acylamino acids, fatty acids having 12 to 24 carbon atoms, alkyl ether carboxylic acids, diacyl glutamic acid lysine, alkylsulfosuccinic acids, N-acyl methyl taurine, alkyl sulfates, polyoxyethylene alkyl ether sulfuric acids, polyoxyethylene alkyl ether phosphoric acids, and salts thereof, (B) a cationic surfactant comprising one or more selected from the group consisting of monoalkyl quaternary ammonium salts, dialkyl quaternary ammonium salts, diacylethyl hydroxyethylmonium methosulfates, and acyl arginine ethyl pyrrolidone carboxylic acid salts, (C) a ceramide, and (D) water, wherein the mass ratio of Component (A) to Component (B), A/B, is 0.5 or more and 60 or less.

2. The emulsified composition according to claim 1, wherein the mass ratio of the total amount of Component (A) and Component (B) to the amount of Component (C), (A+B)/C, is 0.002 or more and 5 or less.

3. The emulsified composition according to claim 1 or 2, further comprising (E) one or more selected from the group consisting of cholesterol and fatty acid cholesterol esters.

4. The emulsified composition according to any one of claims 1 to 3, wherein the anionic surfactant (A) comprises one or more selected from the group consisting of N-acylamino acids and salts thereof.

5. The emulsified composition according to any one of claims 1 to 4, wherein the cationic surfactant (B) comprises one or more selected from the group consisting of mono C12-C24 alkyltrimethylammonium salts, di C12-C24 alkyldimethylammonium salts, diacylethyl hydroxyethylmonium methosulfates, and acyl arginine ethyl pyrrolidone carboxylic acid salts.

6. The emulsified composition according to any one of claims 1 to 5, wherein the content of Component (B) is 0.001 mass% or more and 0.5 mass% or less.

7. The emulsified composition according to any one of claims 1 to 6, wherein the ceramide (C) comprises one or more selected from the group consisting of natural ceramides and pseudo-ceramides.

8. The emulsified composition according to any one of claims 1 to 7, wherein the content of Component (C) is 0.0005 mass% or more and 50 mass% or less.

9. The emulsified composition according to any one of claims 1 to 8, wherein the mass ratio of Component (A) to Component (B), A/B, is 0.75 or more and 40 or less.

10. The emulsified composition according to any one of claims 1 to 9, further comprising (F) a base and/or (G) an acid.

11. The emulsified composition according to any one of claims 1 to 10, wherein the emulsified composition is a cosmetic product.

12. A method for producing the emulsified composition according to claim 10 or 11, comprising:
Step 1 of heating and mixing an oil phase comprising Components (A) to (C);
Step 12 of dissolving the base as Component (F) or the acid as Component (G) in a part of water as Component (D) to obtain an aqueous solution and adding the aqueous solution to the oil phase to obtain a mixture;
Step 13 of cooling the mixture after Step 12; and
Step 2 of mixing an aqueous phase comprising remaining of Component (D) with the oil phase and stirring the resultant.

13. The method for producing the emulsified composition according to claim 12, wherein the oil phase in Step 1 further comprises (E) one or more selected from the group consisting of cholesterol and fatty acid cholesterol esters, and the oil phase comprising Components (A) to (C) and Component (E) is heated and mixed in Step 1.

## Patentansprüche

1. Emulgierte Zusammensetzung, umfassend (A) 0,01 Massen-% oder mehr und 1 Massen-% oder weniger eines anionischen Tensids, das eines oder mehrere, ausgewählt aus der Gruppe, bestehend aus N-Acylaminosäuren, Fettsäuren mit 12 bis 24 Kohlenstoffatomen, Alkylethercarbonsäuren, Diacylglutaminsäurelysin, Alkylsulfobernsteinsäuren, N-Acylmethyltaurin, Alkylsulfaten, Polyoxyethylenalkylether-Schwefelsäuren, Polyoxyethylenalkylether-Phosphorsäuren und Salzen davon, umfasst, (B) ein kationisches Tensid, das eines oder mehrere, ausgewählt aus der Gruppe, bestehend aus quartären Monoalkylammoniumsalzen, quartären Dialkylammoniumsalzen, Diacylethylhydroxyethylmoniummethosulfaten und Acylargininethylpyrrolidoncarbonsäuresalzen, umfasst, (C) ein Ceramid und (D) Wasser, wobei das Massenverhältnis von Komponente (A) zu Komponente (B), A/B, 0,5 oder mehr und 60 oder weniger beträgt.

2. Emulgierte Zusammensetzung gemäß Anspruch 1, wobei das Massenverhältnis von der Gesamtmenge von Komponente (A) und Komponente (B) zu der Menge der Komponente (C), (A+B)/C, 0,002 oder mehr und 5 oder weniger beträgt.

3. Emulgierte Zusammensetzung gemäß Anspruch 1 oder 2, ferner umfassend (E) eines oder mehrere, ausgewählt aus der Gruppe, bestehend aus Cholesterin und Fettsäurecholesterinestern.

4. Emulgierte Zusammensetzung gemäß einem der Ansprüche 1 bis 3, wobei das anionische Tensid (A) eines oder mehrere, ausgewählt aus der Gruppe, bestehend aus N-Acylaminosäuren und Salzen davon, umfasst.

5. Emulgierte Zusammensetzung gemäß einem der Ansprüche 1 bis 4, wobei das kationische Tensid (B) eines oder mehrere, ausgewählt aus der Gruppe, bestehend aus Mono-C12-C24-alkyltrimethylammoniumsalzen, Di-C12-C24-Alkyldimethylammoniumsalzen, Diacylethylhydroxyethylmoniummethosulfaten und Acylargininethylpyrrolidoncarbonsäuresalzen, umfasst.

6. Emulgierte Zusammensetzung gemäß einem der Ansprüche 1 bis 5, wobei der Gehalt der Komponente (B) 0,001 Massen-% oder mehr und 0,5 Massen-% oder weniger beträgt.

7. Emulgierte Zusammensetzung gemäß einem der Ansprüche 1 bis 6, wobei das Ceramid (C) eines oder mehrere, ausgewählt aus der Gruppe, bestehend aus natürlichen Ceramiden und Pseudo-Ceramiden, umfasst.

8. Emulgierte Zusammensetzung gemäß einem der Ansprüche 1 bis 7, wobei der Gehalt der Komponente (C) 0,0005 Massen-% oder mehr und 50 Massen-% oder weniger beträgt.

9. Emulgierte Zusammensetzung gemäß einem der Ansprüche 1 bis 8, wobei das Massenverhältnis von Komponente (A) zu Komponente (B), A/B, 0,75 oder mehr und 40 oder weniger beträgt.

10. Emulgierte Zusammensetzung gemäß einem der Ansprüche 1 bis 9, ferner umfassend (F) eine Base und/oder (G) eine Säure.

11. Emulgierte Zusammensetzung gemäß einem der Ansprüche 1 bis 10, wobei die emulgierte Zusammensetzung ein kosmetisches Produkt ist.

12. Verfahren zur Herstellung der emulgierten Zusammensetzung gemäß Anspruch 10 oder 11, umfassend:
Schritt 1 des Erwärmens und Mischens einer Ölphase, umfassend die Komponenten (A) bis (C);
Schritt 12 des Lösens der Base als Komponente (F) oder der Säure als Komponente (G) in einem Teil des Wassers als Komponente (D), um eine wässrige Lösung zu erhalten, und des Hinzufügens der wässrigen Lösung zu der Ölphase, um eine Mischung zu erhalten;
Schritt 13 des Abkühlens der Mischung nach Schritt 12; und
Schritt 2 des Mischens einer wässrigen Phase, die den Rest der Komponente (D) umfasst, mit der Ölphase und des Rührens des Resultierenden.

13. Verfahren zur Herstellung der emulgierten Zusammensetzung gemäß Anspruch 12, wobei die Ölphase in Schritt 1 ferner (E) eines oder mehrere, ausgewählt aus der Gruppe, bestehend aus Cholesterin und Fettsäurecholesterinestern, umfasst und die Ölphase, umfassend die Komponenten (A) bis (C) und Komponente (E), in Schritt 1 erhitzt und gemischt wird.

## Revendications

1. Composition émulsifiée comprenant (A) 0,01 % en masse ou plus et 1 % en masse ou moins d'un tensioactif anionique comprenant un ou plusieurs choisis dans le groupe consistant en acides N-acylaminés, acides gras présentant 12 à 24 atomes de carbone, acides alkyl éther carboxyliques, acide diacyl glutamique lysine, acides alkylsulfosucciniques, N-acyl méthyl taurine, sulfates d'alkyle, acides polyoxyéthylène alkyl éther sulfuriques, acides polyoxyéthylène alkyl éther phosphoriques, et sels de ceux-ci, (B) un tensioactif cationique comprenant un ou plusieurs choisis dans le groupe consistant en sels d'ammonium quaternaire de monoalkyle, sels d'ammonium quaternaire de dialkyle, méthosulfates d'hydroxyéthylmonium de diacyléthyle, et sels d'acide acyl arginine éthyl pyrrolidone carboxylique, (C) un céramide, et (D) de l'eau, dans laquelle le rapport en masse du Composant (A) au Composant (B), A/B, est de 0,5 ou plus et de 60 ou moins.

2. Composition émulsifiée selon la revendication 1, dans laquelle le rapport en masse de la quantité totale de Composant (A) et de Composant (B) à la quantité de Composant (C), (A+B)/C, est de 0,002 ou plus et de 5 ou moins.

3. Composition émulsifiée selon la revendication 1 ou la revendication 2, comprenant en outre (E) un ou plusieurs choisis dans le groupe consistant en cholestérol et esters de cholestérol d'acides gras.

4. Composition émulsifiée selon l'une quelconque des revendications 1 à 3, dans laquelle le tensioactif anionique (A) comprend un ou plusieurs choisis dans le groupe consistant en acides N-acylaminés et sels de ceux-ci.

5. Composition émulsifiée selon l'une quelconque des revendications 1 à 4, dans laquelle le tensioactif cationique (B) comprend un ou plusieurs choisis dans le groupe consistant en sels de mono alkyltriméthylammonium en C12-C24, sels de di alkyldiméthylammonium en C12-C24, méthosulfates d'hydroxyéthylmonium de diacyléthyle, et sels d'acide acyl arginine éthyl pyrrolidone carboxylique.

6. Composition émulsifiée selon l'une quelconque des revendications 1 à 5, dans laquelle la teneur en Composant (B) est de 0,001 % en masse ou plus et de 0,5 % en masse ou moins.

7. Composition émulsifiée selon l'une quelconque des revendications 1 à 6, dans laquelle le céramide (C) comprend un ou plusieurs choisis dans le groupe consistant en céramides naturels et pseudo-céramides.

8. Composition émulsifiée selon l'une quelconque des revendications 1 à 7, dans laquelle la teneur en Composant (C) est de 0,0005 % en masse ou plus et de 50 % en masse ou moins.

9. Composition émulsifiée selon l'une quelconque des revendications 1 à 8, dans laquelle le rapport en masse du Composant (A) au Composant (B), A/B, est de 0,75 ou plus et de 40 ou moins.

10. Composition émulsifiée selon l'une quelconque des revendications 1 à 9, comprenant en outre (F) une base et/ou (G) un acide.

11. Composition émulsifiée selon l'une quelconque des revendications 1 à 10, dans laquelle la composition émulsifiée est un produit cosmétique.

12. Procédé de production de la composition émulsifiée selon la revendication 10 ou la revendication 11, comprenant :
Étape 1 de chauffage et de mélange d'une phase huileuse comprenant les Composants (A) à (C) ;
Étape 12 de dissolution de la base en tant que Composant (F) ou de l'acide en tant que Composant (G) dans une partie d'eau en tant que Composant (D) pour obtenir une solution aqueuse et ajout de la solution aqueuse à la phase huileuse pour obtenir un mélange ;
Étape 13 de refroidissement du mélange après l'Étape 12 ; et
Étape 2 de mélange d'une phase aqueuse comprenant le reste du Composant (D) avec la phase huileuse et d'agitation de la résultante.

13. Procédé de production de la composition émulsifiée selon la revendication 12, dans lequel la phase huileuse dans l'Étape 1 comprend en outre (E) un ou plusieurs choisis dans le groupe consistant en cholestérol et esters de cholestérol d'acide gras, et la phase huileuse comprenant les Composants (A) à (C) et le Composant (E) est chauffée et mélangée dans l'Étape 1.
